(19) Europäisches Patentamt
European Patent Office
Office européen des brevets

(11) **EP 3 091 349 A1**

(12) **EUROPEAN PATENT APPLICATION**

(43) Date of publication:
**09.11.2016 Bulletin 2016/45**

(51) Int Cl.:
*G01N 21/27* (2006.01)          *G01N 21/78* (2006.01)
*A61K 8/00* (2006.01)           *A61Q 5/00* (2006.01)
*A45D 44/00* (2006.01)          *G01J 3/46* (2006.01)
*A61B 5/00* (2006.01)

(21) Application number: **15166883.7**

(22) Date of filing: **08.05.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(71) Applicant: **The Procter & Gamble Company
Cincinnati, OH 45202 (US)**

(72) Inventors:
• **Eastwood, Ian Michael
  Harrogate, HG2 8QR (GB)**
• **Godfrey, Lynsey Clare
  65824 Schwalbach (DE)**

(74) Representative: **Kremer, Véronique Marie
  Joséphine et al
  Procter & Gamble Service GmbH
  IP Department
  Frankfurter Strasse 145
  61476 Kronberg im Taunus (DE)**

(54) **METHOD FOR THE QUALITY CONTROL OF A COSMETIC COMPOSITION WITH DYEING PROPERTIES**

(57)     A method for the quality control of a cosmetic composition changing its electromagnetic spectral properties under application conditions from an initial state to an end state. The composition is subjected to a measurement condition under which the change of electromagnetic spectral properties occurs over time, the change of electromagnetic spectral properties is measured at least at two different points in time, where the electromagnetic spectral properties at an earlier point in time are different from the electromagnetic spectral properties at the later point in time and at least one value of a parameter derivable from the measured change of electromagnetic spectral properties is determined and compared to a control value of the same parameter.

Fig. 1

EP 3 091 349 A1

**Description**

FIELD OF THE INVENTION

The present invention relates to a method for the quality control of a cosmetic composition changing its electromagnetic spectral properties (hereinafter: ESP) under application conditions, as well as to a method for producing such a composition.

BACKGROUND OF THE INVENTION

The present invention relates to a method for the quality control of a cosmetic composition which changes its ESP under application conditions from an initial state to an end state. The composition is subjected to a measurement condition under which the change of spectral properties occurs over time. Thereby, the change of spectral properties is measured at least at two different points in time, whereof at least one value of a parameter is determined and compared to a control value of the same parameter.

The present invention further relates to a method for the production of a cosmetic composition which changes its ESP under application conditions from an initial state to an end state, wherein a cosmetic composition is produced by mixing at least two constituents, and at least one of the constituents or the mixture or both are subjected to the method for the quality control of the cosmetic composition.

Cosmetic compositions can be used to impart temporary superficial colorization of a surface of the human body, e.g., by providing a colored coating to a body part. Examples for such temporary colorizations are, e.g., nail lacquer, lipstick, eye shadow, and the like. All of these cosmetic compositions can be removed by applying suitable removal methods, e.g., washing with water, organic solvents or other suitable and proven removal compositions. It has, however, also been a long standing practice in human existence to impart a long lasting and durable, non-removable colorization to the human body, which not only changes the appearance of the respective body part by a superficial coating, but also inherently changes the appearance of the respective surface by chemically linking a colorant to a body part. Generally such chemical reactions are performed only on keratinous parts of the body, as they usually do not involve cells which are able to perform cell division functions and are thus often called "dead cells". In most cases such a permanent colorization is performed by applying a dyeing material on skin appendages, such as hair or nails. Especially the permanent colorization of hair has been the subject of a plethora of publications for many years, as it significantly contributes to the need of human beings to alter their appearance. While the motivation for such an alteration can be of a temporary nature, e.g., spurred by a temporary need for a different look, most of the permanent colorizations, especially in the field of hair colorization, result from the need to cover natural changes occurring over time, e.g., the natural change of hair color with age.

The permanent alteration of the hair color by the application of hair dyes is well known. In order to provide the consumer with the shade and the intensity of color desired, a complex chemical process is utilized. Permanent hair dyeing formulations typically comprise oxidative hair dye precursors, which can diffuse into the hair through the cuticle and into the cortex where they then react with each other and a suitable oxidizing agent to form the end dye molecules. Due to their larger size, the resultant molecules are unable to readily diffuse out of the hair during subsequent washing with water and/or detergents; hence delivering a consumer-desired permanency of color. This reaction typically takes place in an aggressive environment at approximately pH 10 in the presence of an alkalizing agent and an oxidizing agent. Typically an oxidizing composition (also called developer and/or oxidizing component) comprising the oxidizing agent and a dye composition (also called tint or dye component) comprising the alkalizing agent and if present the precursors dye molecules are mixed shortly before use. The consumer repeats this process regularly in order to maintain the desired hair color and shade and the intensity of color and to ensure continual, even coverage of the hair including coverage of new hair growth.

It is obvious that any alteration of the appearance of a human being will be subject to the demand for a predictable and reproducible result when applied to the person. Especially in situations when a colorization is applied in order to provide for a continuous outer appearance, there is a need for a rigorous quality control of the dyeing material in order to provide for a possibility to colorize with a dye in exactly the same color and hue as in the previous dyeing step with the same kind of dye. Such a requirement becomes especially obvious when considering the dyeing of growing hair, which, especially for greying hair, is applied to parts of the hair which has just freshly grown and often can form a noticeable differently colored line at spots where hair is parted. It has been a long standing problem to provide hair dyeing colorizations or hair dyes which can exactly reproduce the color of pre colored hair at those freshly grown parts, which are often called "hair roots", despite the fact that they are not "roots" in a biological sense but simply the most recent parts of the freshly grown hair.

It is imperative that such hair dyeing formulations must be subject to high quality standards. The composition of the ingredients of any such hair dyeing formulation has to be most accurate, in order to allow for a most accurate reproduction of a color shade or hue that has been applied before. It is thus important that every product of the same hair dyeing formulation, i.e., of the cosmetic composition, has to match the composition of the one previously applied.

In order to fulfill the high quality standards of cosmetic compositions such as hair dyeing (or colouring) compositions, the quality of the compositions has to be controlled during their manufacture. As the quality of a cosmetic composition with dyeing properties is dependent on various aspects, likewise, the quality control itself involves various aspects. One aspect for controlling the quality of a dyeing cosmetic composition is to control its chemical composition by way of assessing the development of the color.

Up to the present, methods for assessing the dyeing properties of a cosmetic product are usually carried out by a visual inspection of the dyeing process by a human operator. It is evident that such a visual inspection process is prone to many individual and systematical errors, which can result in a dyeing composition not fulfilling requirements as set out above. It will further be understood that such human operated inspection processes are not fit to be performed in modern line production processes, as they usually are very slow and time consuming.

For example, in a current method for assessing the color performance of hair color products the dyeing composition of a production batch to be assessed is applied to a multi-fiber cloth sample or hair swatch (standard hair strands) and visually compared to a sample of the last known good production batch. According to this method a multi-fiber cloth sample or standardized strand of hair is subjected to a complete dyeing process including dyeing, rinsing and drying, which not only takes a significant amount of time, but also bears many variables influencing the result.

One major problem is, however, that the method of assessment is solely dependent on a human operator's visual assessment. That is, the method is highly subjective. Whether a product passes or fails the quality control may differ from operator to operator. Even for one and the same operator the last known good production batch may differ from batch to batch, as it is impossible for a human operator to identify slight changes of a color shade. With such methods a quality drift of the product is almost inevitable.

A further problem resulting from the necessity of a human operator, is that the method is difficult to integrate into a continuous line production manufacturing environment, since the method is very time-consuming. Not only the dyeing and inspection process is time consuming, but also a notable amount of time has to be spent for the preparation of the dye samples. Consequently, a continuous assessment of product quality is almost impossible, since the result is only available with a considerable delay. While this finding may still be acceptable for batch processes, though it also delays the release of a batch for portioning, it is completely inacceptable for continuous production processes.

A further problem of the prior art methods is that they give only qualitative results. It may thus only be assessed whether a production batch passes or fails the quality control. However, the source of a failed production batch may not be detected. Still worse, a production batch may pass the quality control when fulfilling the visual requirements, while not corresponding to the quality standard regarding the ingredients. That is, the quality of the production batch with respect to the composition of its ingredients may not be controlled. Even an absence of one or more specific ingredients of the production batch to be assessed may not be detected.

There is thus a need for a method for the control of a cosmetic composition which changes its ESP under application conditions, which is fast, accurate, runs reliably in a manufacturing environment, enables a quick release of product from a plant, and allows a quantitative assessment, such that the consumer and/or stylist experiences a more accurate in-use product performance.

EP 1942343 A describes a method for determining the amount of dye present in dyed engine oils by quantitative analysis. The method uses imaging techniques, such as spectrophotometry, and a combination of dilution and standard addition techniques to quantitatively determine the concentration of dye in the engine oil to ascertain whether dye concentrations in engine oil have decreased as compared with original specifications, and to quantify the amount of dye to be added to the dyed engine oil to bring dye concentrations to original concentrations.

WO 08124178 A1 describes methods and devices for in-situ measurement of pigment components of interest during manufacturing of pigment or dye solutions. A sample is irradiated with UV/visible light, and generates an absorbance profile correlated with pigment types and concentrations, as well as color properties of pigments in the sample.

The methods according to the prior art allow a quantitative determination of the concentration of a specific dye in a specific composition by spectrometric methods. However, these methods do not enable quality control of dyeing cosmetic compositions. The quality of such a cosmetic composition is not only dependent on the concentration of a specific dye, but on various parameters, such as pH value, interference of other dyes present in the composition, and number and type of further ingredients.

It was thus an object of the present invention to find a method which enables the control of the quality of colored cosmetic compositions with respect to the composition of their ingredients, as well as to their accurate shade. The method should be fast and accurate, run reliably in a manufacturing environment, enable a quick release of product from the plant, and allow a quantitative assessment, such that the consumer and/or stylist may rely on an accurate in-use product performance.

A quantitative method has been found which enables control of a cosmetic composition of a specific preparation, which changes its ESP under application conditions from an initial state to an end state by statistically comparing specific parameters of the cosmetic composition with the parameters of a database of compositions exhibiting the desired quality.

SUMMARY OF THE INVENTION

The present invention relates to a method for the control of a cosmetic composition which changes its ESP under application conditions from an initial state to an end state, wherein

- the composition is subjected to a measurement condition under which the change of spectral properties occurs over time,
- the change of spectral properties is measured at least at two different points in time, where the spectral properties at an earlier point in time are different from the spectral properties at the later point in time,
- at least one value of a parameter derivable from the measured change in spectral properties is determined, and compared to a control value of the same parameter.

Further, the present invention relates to a method for the production of a cosmetic composition which changes its ESP under application conditions from an initial state to an end state, wherein the cosmetic composition is produced by mixing at least two constituents and at least one of the constituents or the mixture or both is subjected to the method according to the invention.

BRIEF DESCRIPTION OF THE DRAWINGS

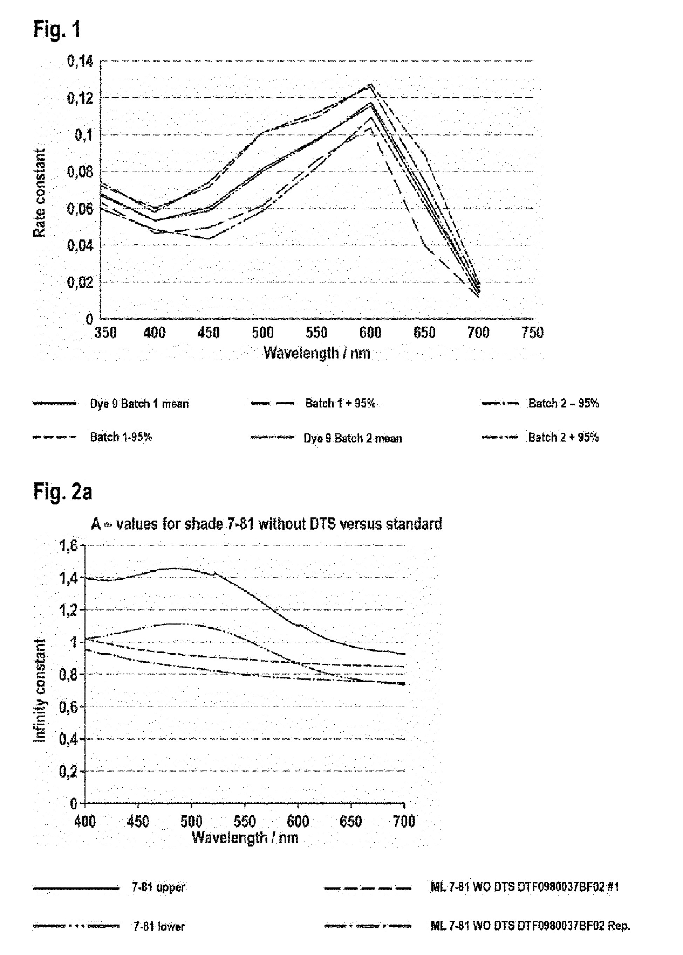

Figure 1 is a graphic representation of the results described for the comparative measurements of two different batches of the same hair dye. The mean values for both measurements are shown, as well as the lines for +95% and -95% confidence intervals.

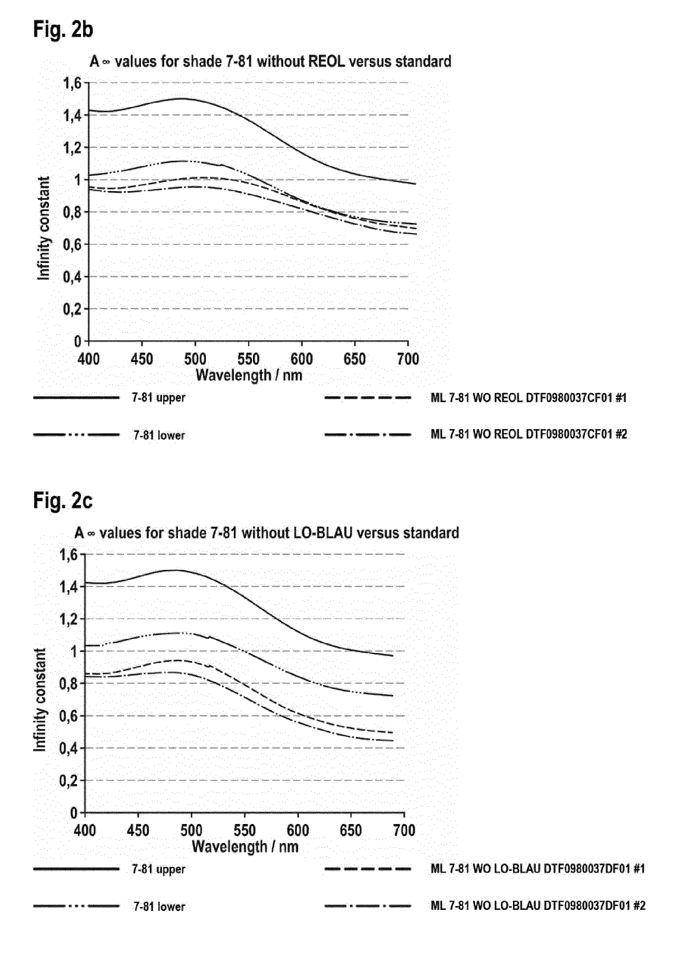

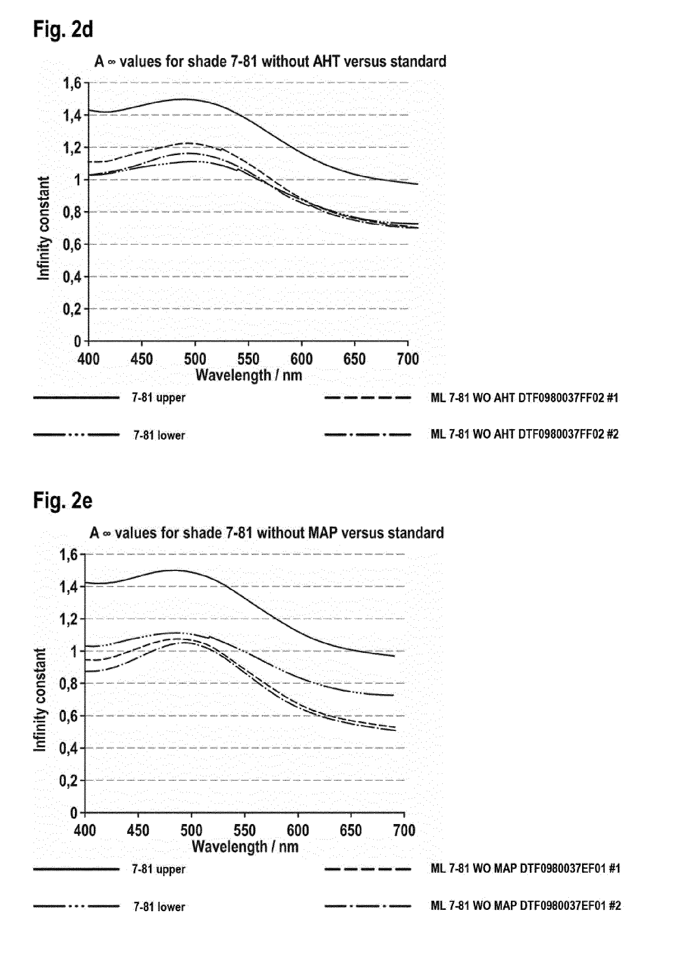

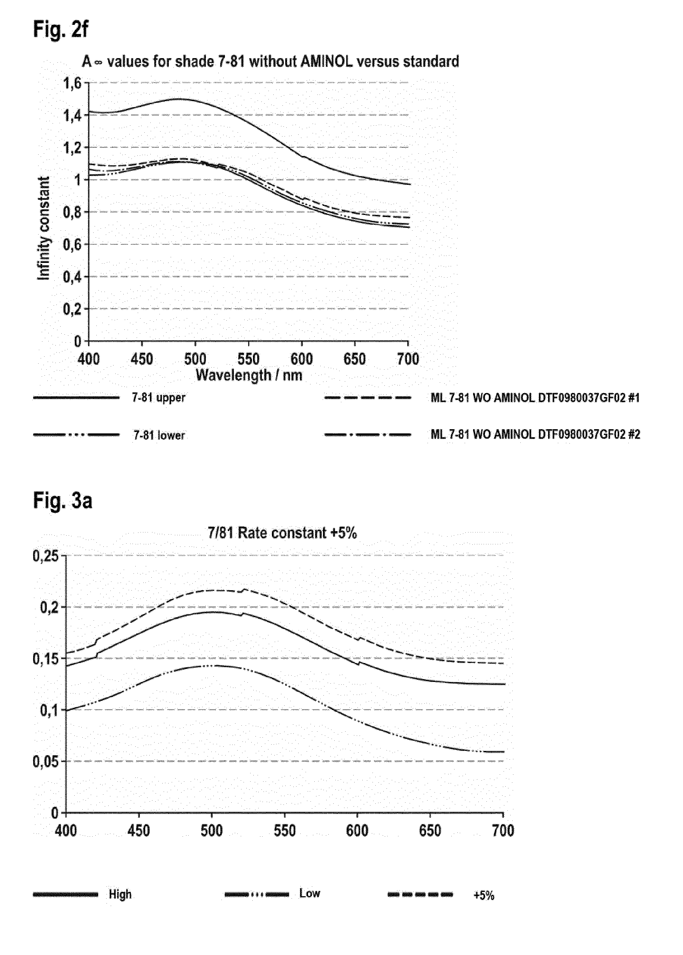

Figures 2a to 2f show a graphic representation of the results obtained for comparative measurements of the commercially available product Wella Illumina Color 7/81. For each measurement the product Wella Illumina Color 7/81 was compared to a batch of the same general composition but missing one of the ingredients needed for proper colorization of the hair. It is immediately obvious that all of the compositions missing an ingredient fail the test according to the claimed method, as the results obtained lie at least partially outside of the range determined for the complete composition.

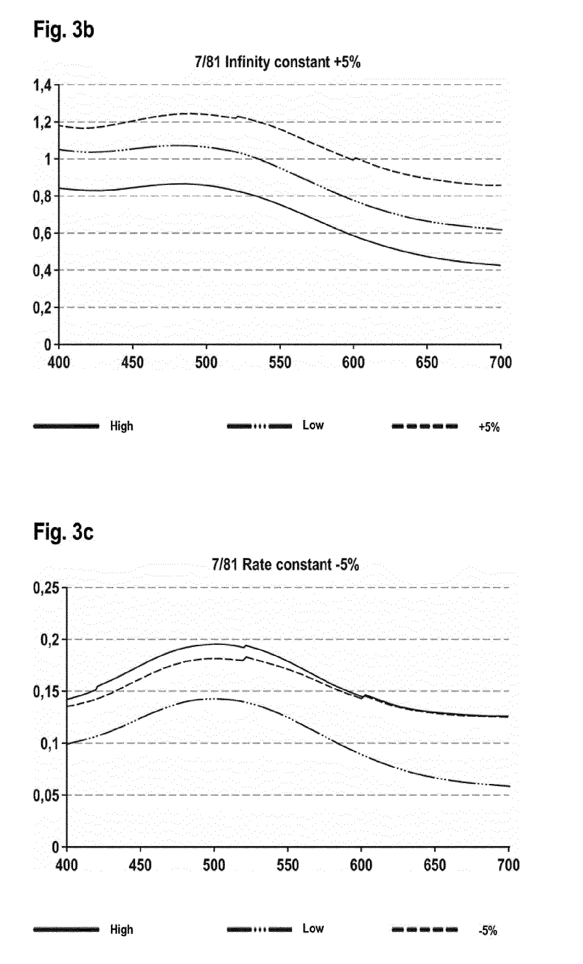

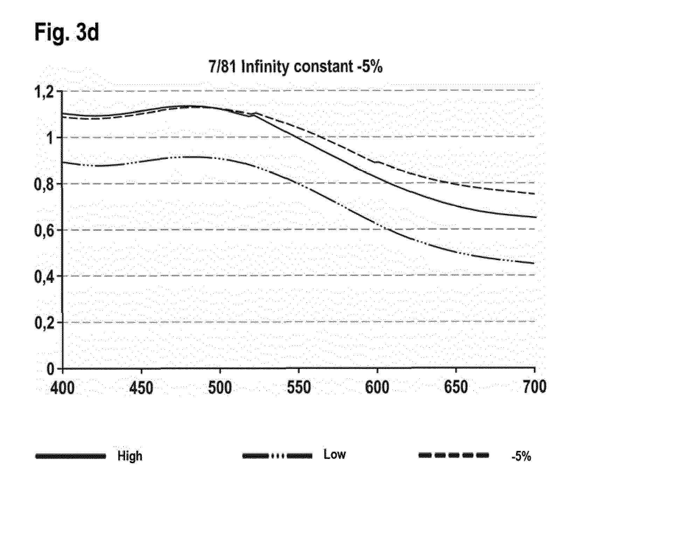

Figures 3a to 3d show a graphic representation of measurement results obtained for comparative measurements of the commercially available product Wella Illumina Color 7/81. For each of the measurements a sample of commercially available product Wella Illumina Color 7/81 was compared to a batch of a sample with the same ingredients where, however, the amount of one of the key dye components was increased by 5% (3a and 3b) or reduced by 5% (3c and 3d) as compared to the sample of the commercially available material.

DETAILED DESCRIPTION OF THE INVENTION

The method according to the present invention relates to the control of a cosmetic composition. The term "control" in the context of the present invention is to be understood as a review of specific aspects of the composition of a product. The control of a cosmetic composition according to the present invention especially relates to the control of the ability of the composition to impart a permanent or at least semi-permanent color to a part of the body, especially a keratinous part of the body, which is different than the color of the body part before application of the cosmetic composition. It can be preferred that the color change results in a darker color of the respective body part, e.g., a brown hue or a black hue. However, a cosmetic composition to be controlled according to the present invention can also impart a completely different color to the body part, e.g., a red, green or blue hue.
The color imparted by the cosmetic composition to be controlled according to the present invention is permanent or at least semi-permanent. Generally, a colorization of a specific body part is then permanent or semi-permanent, if it cannot be removed by a simple method such as rinsing with water or a solvent, or mechanical cleaning action. Often semi-permanent colorizations impart the desired color for at least one week, often more, e.g., two, three, four or more weeks, even one or more months. A permanent colorization generally colorizes the body part for a longer time. Often, especially in case of keratinous body parts which involve dead cells, the colorization lasts until the colorized cells are lost due to natural replacement by the body. The latter process is especially important for hair, which generally is colored with the aim to impart the color until the hair is lost, be it due to cutting or natural processes.
In the present invention the product to be controlled is a cosmetic composition. Cosmetic compositions according to the present invention may be any substance or a mixture of one or more substance suitable for cosmetic use, i.e.

suitable as body care or hair care. The cosmetic compositions to be controlled according to the present invention are able to impart a different color to a body part, especially a keratinous body part, such as skin appendages. In a preferred embodiment of the invention the cosmetic composition is a hair care composition, e.g., a hair dyeing composition or hair coloring composition.

Cosmetic compositions to be controlled according to the present invention change their ESP under application conditions. The ESP of a cosmetic composition are to be understood as the properties of a composition under specific conditions which result from interaction of the composition with electromagnetic radiation at a given time. Such ESP may be e.g. the absorbance, the transmission, the scattering, the bending, or the reflection of the electromagnetic radiation. It has proven to be advantageous if the electromagnetic property is the absorbance.

Generally, the majority of cases where the present invention is advantageously applied, are cosmetic compositions which are able to colorize human hair. Since such a colorization generally involves a change of the absorbance in the visible light spectrum, the control of ESP is the control of the change of absorbance in the wavelength region of UV- and visible light, i.e., in the region of about 200 to about 750 nm. The term "visible region of light" relates to a part of the electromagnetic spectrum which generally is available to the human eye for the processing of visual information, i.e., to a wavelength region of about 390 to about 700 nm.

As used herein the term "hair" to be treated may be "living" i.e. on a living body or may be "non-living" i.e. in a wig, hairpiece or other aggregation of non-living keratinous fibers. Mammalian, preferably human hair is preferred. However wool, fur and other keratin containing fibers are suitable substrates for the compositions according to the present invention.

By "hair dyeing" or "hair coloring" composition it is meant a composition suitable for changing the color of hair. A hair dyeing or hair coloring composition is referred hereinafter mostly as "the cosmetic composition" or "the composition", unless otherwise specified. A hair dyeing composition or hair coloring composition (both terms are used synonymous throughout the text) comprises oxidative dye precursors or even no, or substantially no, dyes in case of compositions giving the hair a lighter color, such as bleaching compositions, where the change of color is mainly caused by the degradation of the natural melanin contained in the hair shaft by the oxidizing agent. The terms "hair dyeing composition or hair coloring composition" as used herein cover compositions which result in a lighter color shade of the hair as well as such compositions which give the hair a darker color shade, such as hair bleaching and oxidative hair dyeing products.

All percentages given in the text are by weight of the hair coloring composition, i.e. of the ready-to-use composition, unless otherwise specified. When more than one composition are used during a treatment, the total weight to be considered is the total weight of all the compositions applied on the hair simultaneously (i.e. the weight found "on head"), typically resulting from mixing an oxidative composition (also called developer and/or oxidizing composition/component) with a dye composition (also called tint, and/or dye composition/component), unless otherwise specified. All ratios or percentages are weight ratios or weight percentages unless specifically stated otherwise.

The change of ESP of a cosmetic composition according to the invention occurs under application conditions. This means that the cosmetic composition is generally present, e.g., stored, in a state where no noticeable change of electromagnetic properties occurs within a longtimeframe, e.g., within about a month, or two or three months or longer, e.g., about a year or two years or more. The change of ESP which is to be controlled according to the invention, occurs under specific conditions which are attributable to the application of the cosmetic composition for its intended use. In case of compositions which impart color to a body part, especially to hair, such application conditions involve chemical reactions of constituents of the cosmetic composition. The term "application conditions" thus relates to conditions under which the cosmetic composition is intended to be used, i.e., conditions which generally occur with the dyeing of hair and the like. Specifically, application conditions are conditions where a chemical reaction of constituents of the cosmetic composition is initiated, which results in a change of color of the cosmetic composition that can be monitored according to the invention.

The change of ESP generally occurs such that the change is observable starting from an initial state and ending at an end state. The initial state is the state of the composition at the moment of initiation of a reaction resulting in the change of ESP. As this reaction is generally a chemical reaction, it may be that the initial state is observable over an extended period of time, such that it may not be necessary to immediately start monitoring the change of ESP with the start of the reaction, but monitoring can be delayed for a certain amount of time until the change of ESP becomes detectable.

As it may often be that application conditions result in a comparatively slow change of ESP, it has proven to be advantageous if for the monitoring and control purposes of the present invention, the cosmetic composition is subjected to a measurement condition under which the change of spectral properties occurs over time. The measurement condition can be the same as the application condition, but it is equally possible that a measurement condition is chosen such that the change of ESP occurs faster or according to a different reaction scheme or different reaction kinetics as compared to application conditions. Such measurement conditions can involve the use of additional compounds in order to influence reaction time, type or kinetics in general, it can involve the addition of solvents, elevated temperatures and the like. Generally, measurement conditions are chosen such that the change of ESP occurs faster than under application conditions, i.e., faster by a factor of 1.2, or 1.5, or 2 or more, even by a factor of 3, 4, 5, 6 or 10 or more.

The color change of a cosmetic composition to be controlled according to the present invention generally proceeds along a chemical reaction path until it has reached a stable end state. The term "stable end state" designates a state in which the ESP of the composition does not change to a significant amount within a timeframe of about 10 minutes or about 5 minutes or even less, e.g. about 1 minute or about 0.5 minutes or less, i.e., the chemical reaction resulting in the change of ESP has ended, i.e., it has proceeded to an amount of at least 90% or at least 95% or at least 99 % or more, e.g., up to about 100%. Under application conditions the term "end state" must not necessarily describe the same situation as the term "stable end state", since application conditions can require to remove the cosmetic composition from the application site before a "stable end state" is reached. Thus, the term "end state, as used throughout this text, denominates the state at which the interaction between cosmetic composition and the body part to be treated has ceased, be it due to an external influence, e.g., due to a removal of the composition from the body part such as rinsing with water or the like, or be it due to the reaction actually reaching a "stable end state".

According to the present invention, the change of spectral properties is measured at least at two different points in time, where the ESP at an earlier point in time are different from the ESP at the later point in time. In case of hair colorizations, the difference in ESP in the visible light region is often such that the absorbance of visible light in this region increases for a specific wavelength. It can, however, also be that in the above specified wavelength range a region exists, where the absorbance of light increases, while in another region the absorbance decreases at the same time.

According to the invention, at least one value of a parameter derivable from the measured change in ESP is determined and compared to a control value of the same parameter. Generally, the measurement of an ESP results in a number, which then is, by using the laws of physics, being attributed to a physical property of the measured material. Thus, the term "derivable" in the context of the present invention means that at least one value of a parameter derivable from the measured change in ESP is determined and compared to a control value of the same parameter. Generally, the measurement of an ESP results in a number, which then is, by using the laws of physics, being attributed to a physical property of the measured material. Thus, the term "derivable" in the context of the present invention means that a measured value is either taken as such, or put into a physical perspective, be it alone or together with one or more values obtained at the same measurement point or at a different measurement point. As an example, it is possible and well known to the skilled person to calculate reaction kinetic data from the change in absorption of light at specific wavelengths, or to calculate an absorption for a specific wavelength at infinite reaction time, and the like.

The invention is based on the finding that the values obtained according to the above method can be compared to values of the same parameter set obtained for a composition which is known to have desired properties, i.e., properties which enable the product to be sold on the market without risking the product to have hidden detrimental properties, which would not have been discovered using the prior art methods for quality control.

As has been described above, the change of ESP can be measured under any type of condition which allows for the generation of data useful in quality control of a cosmetic composition. In the case of cosmetic compositions with dyeing properties, e.g., in case of hair dyes, it has proven to be successful, if the change of ESP is measured with light in the UV/VIS region, preferably at a wavelength in the region of 200 nm to 700 nm.

Generally, the measurement of a change in ESP involves the change of those properties as a result of a reaction of one or more of the constituents of a composition to be measured with either one or more other components of the composition, or with an external reactant, e.g., oxygen. As the amount of reactants in the composition is limited, it is clear that the reaction proceeds from an initial state, where all of the reactants are still present as educts, to an end state, where all of the reactants have completed the reaction and are present as products.

The term "all of the reactants have completed the reaction" is to be interpreted such that reactions generally proceed to an end state where it is possible that still a very minor amount of the reactants are present in unreacted form, since dilution of the reactants or viscosity effects or both or even other effects can result in a considerable slowing down of the reaction process and overall different reaction kinetics. This effect, however, does not have any influence on the reaction parameters to be measured according to the invention.

It is generally possible according to the invention to measure the change of ESP within a timeframe adapted to the reaction to be measured, e.g., within a timeframe chosen such that the reaction to be measured is completed at least to an amount of 30%, or at least 50%, or at least 60% or at least 70%, or at least 80% or at least 90% or at least 95 % or at least 99%.

According to the invention it can be preferred if the change of ESP from an initial state to an end state under application conditions occurs over a time range from 1 second to 10 hours, e.g., over a time range from 5 seconds to 5 hours or 30 seconds to 2 hours or 1 minute to 1 hour or 10 minutes to 45 minutes.

It can be preferred according to the invention, if the change of electromagnetic spectral properties comprises a color change in the visible region of light.

According to the invention the change of ESP is measured under measurement conditions. Generally, the measurement conditions can be chosen such that they are identical to application conditions. This can be especially preferred if the application conditions allow for a reaction of the composition to be measured which results in a change of ESP within the above mentioned timeframe. However, often it can be preferred if a measurement condition is chosen such that the change of spectral properties is faster than under application conditions, e.g., by a factor of 1.2 or more, or 1.5 or more, or 2 or more, or 3or more, or 4 or more, or 5 or more, or 10 or more.

Accuracy of the measurement can be dependent on the amount of change of the measured property within the timeframe available for measurement. It can thus be preferred, if the ESP to be measured is chosen such that its amount of change is high within a short timeframe. It can be preferred, if the measurement condition is chosen such that the change in ESP occurs such that a wavelength for measurement can be chosen where the amount of change between initial state and stable end state is at least 80%, or at least 90% or at least 95 % or at least 99 % within a timeframe of 1 to 20 minutes, or 2 to 15 minutes or 5 to 12 minutes. It should be noted that it is possible, but not necessary to measure the change of ESP over the essentially complete course of the reaction. It can generally be sufficient to measure the change of ESP over only a fraction of the reaction course, e.g., to measure only until the reaction has proceeded to a completion of about 75% or less, or about 70% or less, or about 60% or less, or about 50% or even less than 45% or less than 40%.

In a preferred embodiment of the present invention, the cosmetic composition is a hair dyeing composition or hair coloring composition.

A hair dyeing composition or hair coloring composition to be tested according to the present invention may comprise oxidative dye precursors, which are usually classified either as primary intermediates (also known as developers) or couplers (also known as secondary intermediates). Various couplers may be used with primary intermediates in order to obtain different shades. Oxidative dye precursors may be free bases or the cosmetically acceptable salts thereof.

Typically, the composition may comprise a total amount of oxidative dye precursors ranging up to about 12%, alternatively from about 0.1% to about 10%, alternatively from about 0.3% to about 8%, alternatively from about 0.5% to about 6%, by weight of the total composition.

Suitable primary intermediates include, but are not limited to: toluene-2,5-diamine, p-phenylenediamine, *N*-phenyl-p-phenylenediamine, *N,N*-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1,2-diaminothane, 2-[(3-aminopyrazolo[1,5-a]pyridin-2-yl)oxy]ethanol hydrochloride, salts thereof and mixtures thereof.

Suitable couplers include, but are not limited to: resorcinol, 4-chlororesorcinol, 2-chlororesorcinol, 2-methylresorcinol, 4,6-dichlorobenzene-1,3-diol, 2,4-dimethylbenzene-1,3-diol, *m*-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 6-hydroxybenzomorpholine, 2-amino-5-ethylphenol, 2-amino-5-phenylphenol, 2-amino-5-methylphenol, 2-amino-6-methylphenol, 2-amino-5-ethoxyphenol, 5-methyl-2-(methylamino)phenol, 2,4-diaminophenoxyethanol, 2-amino-4-hydroxyethylaminoanisole, 1,3-bis-(2,4-diaminophenoxy)-propane, 2,2'-(2-methyl-1,3-phenylene)bis(azanediyl)diethanol, benzene-1,3-diamine, 2,2'-(4,6-diamino-1,3-phenylene)bis(oxy)diethanol, 3-(pyrrolidin-1-yl)aniline, 1-(3-(dimethylamino)phenyl)urea, 1-(3-aminophenyl)urea, 1-naphthol, 2-methyl-1-naphthol, 1,5-naphthalenediol, 2,7-naphthalenediol or 1-acetoxy-2-methylnaphthalene, 4-chloro-2-methylnaphthalen-1-ol, 4-methoxy-2-methylnaphthalen-1-ol, 2,6-dihydroxy-3,4-dimethylpyridine, 2,6-dimethoxy-3,5-pyridinediamine, 3-amino-2-methylamino-6-methoxypyridine, 2-amino-3-hydroxypyridine, 2,6-diaminopyridine, pyridine-2,6-diol, 5,6-dihydroxyindole, 6-hydroxyindole, 5,6-dihydroxyindoline, 3-methyl-1-phenyl-1*H*-pyrazol-5(4*H*)-one, 1,2,4-trihydroxybenzene, 2-(benzo[d][1,3]dioxol-5-ylamino)ethanol (also known as hydroxyethyl-3,4-methylenedioxyaniline), and mixtures thereof.

When the composition of the invention is obtained by mixing a tint composition and a developer composition, the primary intermediates and couplers are usually incorporated into the tint composition.

**Oxidizing agents**

A composition to be measured according to the present invention may further comprise at least one source of an oxidizing agent. Any oxidizing agent known in the art may be used. Preferred oxidizing agents are water-soluble peroxygen oxidizing agents. As used herein, "water-soluble" means that in standard conditions at least about 0.1g, preferably about 1g, more preferably about 10g of the oxidizing agent can be dissolved in 1 liter of deionized water at 25°C. The oxidizing agents are valuable for the initial solubilisation and decolorisation of the melanin (bleaching) and accelerate the oxidation of the oxidative dye precursors (oxidative dyeing) in the hair shaft.

Typically, the composition may comprise a total amount of oxidizing agents ranging from about 0.1% to about 10%, alternatively from about 1% to about 7%, alternatively from about 2% to about 5%, by weight of the total composition.

Suitable water-soluble oxidizing agents include, but are not limited to: inorganic peroxygen materials capable of yielding hydrogen peroxide in an aqueous solution.

Suitable water-soluble peroxygen oxidizing agents include, but are not limited to: hydrogen peroxide; inorganic alkali metal peroxides (such as sodium periodate and sodium peroxide); organic peroxides (such as urea peroxide and melamine peroxide); inorganic perhydrate salt bleaching compounds (such as the alkali metal salts of perborates, percarbonates, perphosphates, persilicates, persulphates and the like); and mixtures thereof. Inorganic perhydrate salts may be incorporated for example as monohydrates, tetrahydrates. Alkyl/aryl peroxides and/or peroxidases may also be used. Mixtures of two or more such oxidizing agents can be used if desired. The oxidizing agents may be provided in aqueous solution or as a powder which is dissolved prior to use.

In a specific embodiment, the composition comprises a water-soluble oxidizing agent selected from the group consisting of hydrogen peroxide, percarbonates (which may be used to provide a source of both oxidizing agent and carbonate ions and or ammonium ions), persulphates, and mixtures thereof.

When the composition of the present invention is obtained by mixing a developer composition and a tint composition prior to use, the oxidizing agent may be present in the developer composition. The developer composition may be based on any desired formulation chassis, including any commercial product, for example an oil-in-water emulsion. Typical developer compositions comprise about 6% or about 9% of the $H_2O_2$ relative to the total weight of the developer composition. A commercial example is the Welloxon® Emulsion with respectively about 6% and about 9% $H_2O_2$, marketed by Wella and comprising as INCI ingredients: Water, $H_2O_2$, Cetearyl Alcohol, Ceteareth-25, Salicylic Acid, Phosphoric Acid, Disodium Phosphate, Etidronic Acid.

The composition according to the present invention may comprise, in addition to the ingredients indicated above, further ingredients in order to further enhance the properties of the composition, as long as these are not excluded by the claims.

Suitable further ingredients include, but not limited to: solvents; oxidizing agents; alkalizing agents; oxidative dye precursors, direct dyes; chelants; radical scavengers; pH modifiers and buffering agents; thickeners and/or rheology modifiers; carbonate ion sources; peroxymonocarbonate ion sources; anionic, cationic, nonionic, amphoteric or zwitterionic surfactants, and mixtures thereof; anionic, cationic, nonionic, amphoteric or zwitterionic polymers, and mixtures thereof; fragrances; enzymes; dispersing agents; peroxide stabilizing agents; antioxidants; natural ingredients (such as proteins, protein compounds, and plant extracts); conditioning agents (such as silicones and cationic polymers); ceramides; preserving agents; opacifiers and pearling agents (such as titanium dioxide and mica); and mixtures thereof.

Suitable further ingredients referred to above, but not specifically described below, are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific adjuvants to achieve a particular purpose or multipurpose. A few of these ingredients are discussed hereinbelow, whose disclosure is of course non-exhaustive.

The composition according to the present invention may further comprise compatible direct dyes, in an amount sufficient to provide additional coloring, particularly with regard to intensity. Typically, the composition may comprise a total amount of direct dyes ranging from about 0.05% to about 4%, by weight of the total composition.

Suitable direct dyes include but are not limited to: Acid dyes such as Acid Yellow 1, Acid Orange 3, Acid Black 1, Acid Black 52, Acid Orange 7, Acid Red 33, Acid Yellow 23, Acid Blue 9, Acid Violet 43, HC Blue 16, Acid Blue 62, Acid Blue 25, Acid Red 4; Basic Dyes such as Basic Brown 17, Basic Red 118, Basic Orange 69, Basic Red 76, Basic Brown 16, Basic Yellow 57, Basic Violet 14, Basic Blue 7, Basic Blue 26, Basic Red 2, Basic Blue 99, Basic Yellow 29, Basic Red 51, Basic Orange 31, Basic Yellow 87, Basic Blue 124, 4-(3-(4-amino-9,10-dioxo-9,10-dihydroanthracen-1-ylamino)propyl)-4-methylmorpholin-4-ium-methylsulfate, (*E*)-1-(2-(4-(4,5-dimethylthiazol-2-yl)diazenyl)phenyl)(ethyl)amino)ethyl)-3-methyl-1H-imidazol-3-ium chloride, (*E*)-4-(2-(4-(dimethylamino)phenyl)diazenyl)-1-methyl-1H-imidazol-3-ium-3-yl)butane-1-sulfonate, (*E*)-4-(4-(2-methyl-2-phenylhydrazono)methyl)pyridinium-1-yl)butane-1-sulfonate, N,N-dimethyl-3-(4-(methylamino)-9,10-dioxo-4a,9,9a,10-tetrahydroanthracen-1-ylamino)-N-propylpropan-1-aminium bromide; Disperse Dyes such as Disperse Red 17, Disperse Violet 1, Disperse Red 15, Disperse Black 9, Disperse Blue 3, Disperse Blue 23, Disperse Blue 377; Nitro Dyes such as 1-(2-(4-nitrophenylamino)ethyl)urea, 2-(4-methyl-2-nitrophenylamino)ethanol, 4-nitrobenzene-1,2-diamine, 2-nitrobenzene-1,4-diamine, Picramic acid, HC Red No. 13, 2,2'-(2-nitro-1,4-phenylene)bis(azanediyl)diethanol, HC Yellow No. 5, HC Red No. 7, HC Blue No.2, HC Yellow No. 4, HC Yellow No. 2, HC Orange No. 1, HC Red No. 1, 2-(4-amino-2-chloro-5-nitrophenylamino)ethanol, HC Red No. 3, 4-amino-3-nitrophenol, 4-(2-hydroxyethylamino)-3-nitrophenol, 2-amino-3-nitrophenol, 2-(3-(methylamino)-4-nitrophenoxy)ethanol, 3-(3-amino-4-nitrophenyl)propane-1,2-diol, HC Yellow No. 11, HC Violet No. 1, HC Orange No. 2, HC Orange No. 3, HC Yellow No. 9, HC Red No. 10, HC Red No. 11, 2-(2-hydroxyethylamino)-4,6-dinitrophenol, HC Blue No. 12, HC Yellow No. 6, HC Yellow No. 12, HC Blue No. 10, HC Yellow No. 7, HC Yellow No. 10, HC Blue No.

9, 2-chloro-6-(ethylamino)-4-nitrophenol, 6-nitropyridine-2,5-diamine, HC Violet No. 2, 2-amino-6-chloro-4-nitrophenol, 4-(3-hydroxypropylamino)-3-nitrophenol, HC Yellow No. 13, 6-nitro-1,2,3,4-tetrahydroquinoxaline, HC Red No. 14, HC Yellow No. 15, HC Yellow No. 14, N2-methyl-6-nitropyridine-2,5-diamine, N1-allyl-2-nitrobenzene-1,4-diamine, HC Red No. 8, HC Green No.1, HC Blue No. 14; Natural dyes such as Annato, Anthocyanin, Beetroot, Carotene, Capsanthin, Lycopene, Chlorophyll, Henna, Indigo, Cochineal; and mixtures thereof.

When the composition is obtained by mixing a tint composition and a developer composition, the direct dyes are usually incorporated into the tint composition.

Generally, any kind of parameter value obtainable by the measurement of a change in ESP of a cosmetic composition can be used to establish a useful comparison with a standard value. Standard values are values obtained with a composition which is known to fulfill the quality standards required for the cosmetic composition to be measured.

As has been described above, the method according to the claimed invention has proven to be most successful for the quality control of dyeing compositions which change the color of a body part, especially the color of hair, by a chemical reaction. As such chemical reactions are generally the source of the effect of the product, control of the chemical reaction is useful as a medium of control of the quality of the product. It has thus proven to be successful, if the at least one value of a parameter derivable from the measured change in spectral properties is a kinetic value or an absorbance value or an inflection value or a mixture of two or more of those values.

While absorbance values A are available throughout the course of the reaction if absorbance is measured, it can be subject to variable conditions and obtaining a specific absorbance value at a specific reaction time t can be difficult, since the starting conditions of the reaction may vary, depending upon operating conditions. However, as the reaction itself should be the same for all compositions of a specific type, it has proven to be successful if absorbance at infinite reaction time ($A_\infty$) is determined. The determination of absorbance at infinite reaction time is, e.g., available from a plot of 1/(absorbance A) vs 1/ (reaction time t). The final value for absorbance $A_\infty$ is given when $t \to \infty$, and $1/t \to 0$. This value is denoted by 1/c or infinity constant.

It can further be possible that this plot of 1/(absorbance A) vs 1/ (reaction time t) shows an inflection point, the determination of which gives a unique result for a specific type of cosmetic composition. The inflection point is determined by an examination of the position of the stationary point on a plot of 1/Abs versus 1/time. For a second order polynomial in the form $y = ax^2 + bx + c$, this occurs when the derivative dy/dx = 0, i.e. where x = -b/2a.

A further valuable parameter is the kinetic rate constant $\chi$, which is available from a plot of absorbance A vs log(reaction time t) as the gradient of the line obtainable from such a plot.

The method according to the invention further comprises the step of comparing the measured and calculated value of the parameter derivable from the measured change in spectral properties with a pass/fail-range for this value obtainable by:

1) determining the value of the at least one parameter derivable from the measured change in spectral properties for a population of a specific cosmetic composition with acceptable application properties,
2) determining a population mean, a standard deviation and a standard error for the value of the at least one parameter derivable from the measured change in spectral properties,
3) defining a lower and an upper boundary between which the standard score of at least one value of a parameter derivable from the measured change in spectral properties to be controlled may range

and determining whether the measured and calculated value of the parameter derivable from the measured change in spectral properties lies within this range or outside.

Generally, the size of the population can be small, e.g., about 2 or about 3 samples or more. However, it has proven to be successful, if the population consists of at least 10 samples.

With regard to compositions which can successfully be compared by measurements of absorbance within the visible light region, it can be preferred if the comparison is carried out in a wavelength range of from 400 to 700 nm at small intervals, e.g., at intervals of about 20 nm or less or about 10 nm or less or about 5 nm or less or about 1 nm or even less.

The comparison is then preferably carried out for at least the value of the reaction rate and the value of the absorbance at infinite time or the value of the reaction rate and the value of the inflection point or the value of the reaction rate and the value of the absorbance at infinite time and the value of the inflection point.

The present invention also relates to a method for the production of a cosmetic composition which changes its electromagnetic spectral properties under application conditions from an initial state to a stable end state, wherein a cosmetic composition is produced by mixing at least two constituents and at least one of the constituents or the mixture or both is subjected to a method as described herein.

The claimed method has the advantage that it can be successfully applied also in such production environments, where the production speed does not allow for time consuming quality control efforts. This is especially important for production processes where mixing of the ingredients and filling are continuous processes and a frequent monitoring of the product quality is important.

The invention will be further illustrated by the following examples.

**Examples**

Devices

- UV-Visible Spectrophotometer: JASCO V-650 UV-Visible spectrophotometer with one-drop SAH-769 accessory and software

- PC with Windows operating system

- Positive displacement pipettes: Gilson M100 pipette (100$\mu$l), Gilson M1000 pipette (1000$\mu$l)

- Air displacement pipettes: Gilson P20 pipette (20$\mu$l), Gilson P200 pipette (200$\mu$l), Gilson P1000 pipette (1000$\mu$l), Gilson P10mL pipette (10ml)

- Mixing equipment: Gilson M1000 positive displacement pipette (1000$\mu$l)

- Vials: 20ml Glass Wheaton scintillation vials (PTFE (not foil) lined caps) (Sigma-Aldrich)

- Database: SQL Server 2012 Express Database & PC running Kinetic Color Matching application

- Stopwatch

- Spatula

Reagents and solutions

- Hydrogen peroxide: Sigma 34.5 - 36.5 % (product code: 18304-1L)
- Iron chloride: Sigma Aldrich 45 % (product code: 12322-2.5L)
- Tint 1: Illumina 7/81 by Wella
- Tint 2: Koleston Perfect & Color Touch by Wella shade 4 and shade 5
- Tint 3: Koleston Perfect Japan & NNE (liquid color) by Wella
- Copper chloride: Sigma Aldrich (product code: 222011-250g)
- Deionized water

Preparation of Equipment

1. The spectrophotometer was connected to the socket, the USB port on the spectrophotometer was connected to a USB port on a PC, and the spectrophotometer was switched on.
2. A lamp calibration check was conducted according to JASCO software manual.
3. Instructions of the software ware followed.
4. After a calibration was completed "Baseline"-menu was exited.

Preparation of stock solutions

*Hydrogen peroxide*

10 ml of hydrogen peroxide were diluted in 90 ml of deionized water to give an approximately 3.5 % hydrogen peroxide solution.

*Iron chloride (1.1mg/L) - catalyst for Illumina 7/81*

1 ml of concentrated iron chloride (45 %) were transferred (with the P1000 pipette) into a 20 ml vial and diluted with 19 ml (P10ml) of deionized water (solution A). Solution A is stable for up to 7 days. Next 2 ml (P10ml) of solution A were transferred into a separate vial, and 18 ml (P10ml) of deionized water were added (solution B). Solution B is stable for up to 3 days. 10 ml (P10ml) of solution B were taken, and 10 ml (P10ML) of deionized water were added (solution C) to give a total dilution of 1:600. 20 ml of solution C are enough to perform 133 tests. This solution is stable for up to 1 day.

*Copper chloride (1mg/g) - catalyst for Koleston Perfect*

1g of copper chloride were weighed into a flask and made up to 1kg using de-ionized water. Using a spatula, the mixture was well stirred until the copper salt has fully dissolved. This resulted in a 1mg/g copper chloride solution which is stable for up to 1 week.

Preparation of samples

*Simple 1*

1. 4.0 ml of deionized water were deposited into a 20 ml glass vial using the P10ml 10 ml pipette.
2. A small amount of tint was discarded from the tube comprising tint 1, in order to ensure that the test is performed on material which represents the bulk. Using the M100 100$\mu$l positive displacement (pd) pipette, 150$\mu$l (2 x 75$\mu$l additions) of the test shade were deposited into the vial containing 4.0ml of deionised water.
3. Using the M1000 1000$\mu$l pd pipette, the tint and the water were gently mixed together for 30 seconds until the mix was homogeneous (the liquid was sucked into the pipette and slowly ejected back into the vial).
4. Into a separate 20ml glass vial, 150$\mu$l of 1.1mg/ml iron chloride solution C (using the P200 200$\mu$l pipette) and 150$\mu$l 3.5% peroxide solution were deposited.
5. The timer was started.
6. Using the P1000 1000$\mu$l pipette, all of the catalyst-peroxide solution of step 4 was added into the shade solution of step 3. Mixing was preformed by sucking the liquid into the pipette and slowly ejecting back into the vial for 5 seconds. Introduction of air bubbles into the system is to be avoided.
7. At approximately 45 seconds after step 4 (the waiting time should ensure that the sample is essentially bubble-free), the P20 20$\mu$l pipette was used to eject 20$\mu$l of the reaction solution of step 6 onto the sensor surface of the one-drop accessory of the spectrophotometer.
8. The device was closed, and the measurement was started.

*Simple 2*

1. The P10ml 10ml pipette (set to 4ml) was used to deposit 4.0 ml of deionized water into a 20ml glass vial.
2. The M100 100$\mu$l positive displacement (pd) pipette was used to deposit 150$\mu$l (2 x 75$\mu$l additions) of tint 2 into the vial containing 4.0ml of deionised water
3. Using the M1000 1000$\mu$l pd pipette, the tint and the water were gently mixed together for 30 seconds until the mixture was homogeneous (the liquid was sucked into the pipette and slowly ejected back into the vial).
4. Into a separate 20ml glass vial, 150$\mu$l of the copper chloride solution and 150$\mu$l of the peroxide solution were deposited (using the P200 200$\mu$l pipette).
5. The timer was started, in order to wait for copper chloride and peroxide solution to react.
6. After 30 seconds, the P1000 1000$\mu$l pipette was used to add all (300$\mu$l) of the catalyst-peroxide solution (4) into the tint solution (3). Quick Mixing with the pipette was conducted for 5 seconds.
7. The timer was started again (20 seconds, the solution is now essentially bubble-free)).
8. The P20 20$\mu$l pipette was used to eject 20$\mu$l of the reaction solution (6) onto the read surface of the one-drop accessory, and the device was closed.
9. At 20 seconds, the measurement was started.

*Sample 3*

1. The P10ml 10ml pipette (set to 8ml) was used to deposit 8.0 ml of deionized water into a 20ml glass vial.

2. The M100 100µl positive displacement (pd) pipette was used to deposit 150µl (2 x 75µl additions) of tint 3 into the vial containing 8.0ml of deionized water.

3. Using the M1000 1000µl pd pipette, the tint and the water were gently mixed together for 30 seconds until the mixture was homogeneous (the liquid was sucked into the pipette and slowly ejected back into the vial).

4. Into a separate 20ml glass vial, 150µl of the copper chloride solution and 150µl of the peroxide solution were deposited (using the P200 200µl pipette, respectively).

5. The timer was started (30 seconds), in order to wait for copper chloride and peroxide solution to react.

6. After 30 seconds, the P1000 1000µl pipette was used to add all (300µl) of the catalyst-peroxide solution (4) into the tint solution (3). Quick Mixing with the pipette was conducted for 5 seconds.

7. The timer was started again (20 seconds, the solution is now essentially bubble-free).

8. The P20 20µl pipette was used to eject 20µl of the reaction solution (6) onto the read surface of the one-drop accessory, and the device was closed.

9. At 20 seconds, the measurement was started.

Instrument operation

1. The spectrophotometer then performs the following actions and calculations:

   a) The absorbance of the dye is then measured each minute for every wavelength, from 400nm to 700nm.
   b) The values obtained for each wavelength are used to calculate the rate reaction by using

$$A = c(1 - e^{-kt}) + d$$

   where:

   A is the absorbance
   c is the maximum absorbance
   k is the rate constant
   t is time
   d is the contribution from direct or pre-reacted dye.

   From a plot of A vs. log (t) for each wavelength a straight line can be obtained which allows to calculate the rate constant k. From a plot of 1/A vs. 1/t the final color (Absorbance A for a given wavelength) is calculated. The final value for absorption is given when the time $t \to \infty$, i.e. when $1/t \to 0$.
   Furthermore, the position of the stationary point on this plot is examined. For a second order polynomial in the form $y = ax^2 + bx + c$ such a stationary point occurs when the derivative $dy/dx = 0$, i.e. where $x = -b/2a$.
   The above calculations can be performed by any software or also by manually calculating the above values.

2. It can be advantageous to use any evaluation software in order to obtain a visual representation of the scanned values as a graph. It can further be advantageous to perform multiple scans, e.g. 10 scans, ion order to have a representative set of values for the development of the absorbance over time for different wavelengths.

3. It can further be advantageous to repeat the testing procedure several times, e.g., three times, in order to find out whether the homogeneity of the sample is satisfactory. Should this not be the case (e.g. due to too many bubbles being present in the solution to be measured), the test can be aborted and a new sample prepared.

*Statistical calculations*

Whether the tint passes or fails the quality control, is determined by calculating a population of samples for each shade, using as many 'passed' batches as possible. The samples are assayed to the method according to the present invention, and the kinetic parameters are calculated as described above. From these data a standard deviation is generated for the three parameters rate constant, absorbance at infinite time and inflection point. Assuming a standard bell shaped distribution for the population, upper and lower bounds have been defined at 99% (2.58 SD) as the pass/fail criterion. Thus, the upper and lower bounds are defined as the average plus or minus 2.58 SD, respectively. The batch will be deemed to have failed if any result falls outside of the upper and lower boundaries for the specified tint.

This statistical comparison is made for the rate constant, A∞ value and inflection point every 1nm from 400 nm to 700nm, i.e. 300 results. Using said three parameters, 900 data points are given for comparison.

Example 1

Koleston Perfect shade 5 (a light brown shade) has been compared to Koleston Perfect shade 4 (a medium brown shade) according to the method described under section "Calculations". This comparison shows that the mere statistical treatment of the results gives a clear indication of the difference between the two shades, though the visual difference is small.

The results are illustrated in Table 1 below.

Table 1: Z values calculated from the rates of reaction of shade 4/ and 5/ (brown shades).

| Wavelength nm | SE | $\overline{\chi_4}$ - $\overline{\chi_5}$ | Z | Test Statistic | | |
| --- | --- | --- | --- | --- | --- | --- |
| | | | | 90 %<br>-1.64 ≤ Z ≤ 1.64 | 95 %<br>-1.96 ≤ Z ≤ 1.96 | 99 %<br>-2.58 ≤ Z ≤ 2.58 |
| 350 | 0.003983 | 0.15485 | 38.8757 | sig diff* | sig diff* | sig diff* |
| 400 | 0.002526 | 0.045725 | 18.09906 | sig diff* | sig diff* | sig diff* |
| 450 | 0.005539 | 0.128938 | 23.27962 | sig diff* | sig diff* | sig diff* |
| 500 | 0.008961 | 0.227363 | 25.37212 | sig diff* | sig diff* | sig diff* |
| 550 | 0.010262 | 0.134825 | 13.13861 | sig diff* | sig diff* | sig diff* |
| 600 | 0.00964 | 0.071175 | 7.383503 | sig diff* | sig diff* | sig diff* |
| 650 | 0.005116 | 0.014325 | 2.799924 | sig diff* | sig diff* | sig diff* |
| 700 | 0.001879 | -0.0245 | -13.0403 | sig diff* | sig diff* | sig diff* |
| *= significant difference | | | | | | |

The results obtained from the measurements have been subjected to a statistical Z-test, wherein with two populations of data whose average is $a_1$ and $a_2$ the standard error (SE) of the mean is represented by:

$$SE = \sqrt{\frac{\sigma_1^2}{n_1} + \frac{\sigma_2^2}{n_2}}$$

and the Z- score (test statistic) is

$$Z = \frac{\chi_4 - \chi_5}{SE}$$

In Table 1 it is clearly shown that the Z value of Koleston Perfect shade 5/ is outside the 99% confidence limits at all wavelengths. That is, Koleston Perfect shade 5/ has correctly failed the quality control according to the present invention, as it has been compared to a different color shade.

Example 2

2 batches of Koleston Perfect shade 9/0 (Koleston Perfect 9a and 9b) have been compared according to the method described under section "Calculations". The results are illustrated in Table 3 below. Fig. 1 further shows the graphical interpretation of the results.

Table 3: Z values calculated from the rates of reaction of two manufacturing lots of shade 9/0.

| Wavelength nm | SE | $\overline{x_{9a}}$ - $\overline{x_{9b}}$ | Z | Test statistic | | |
|---|---|---|---|---|---|---|
| | | | | 90 %<br>$-1.64 \leq Z \leq 1.64$ | 95 %<br>$-1.96 \leq Z \leq 1.96$ | 99 %<br>$-2.58 \leq Z \leq 2.58$ |
| 350 | 0.001523 | 0.000637 | 0.418611 | same | same | same |
| 400 | 0.001496 | 6.25E-05 | 0.041766 | same | same | same |
| 450 | 0.003389 | 0.001788 | 0.52746 | same | same | same |
| 500 | 0.005244 | 0.001363 | 0.259822 | same | same | same |
| 550 | 0.003403 | 0.000637 | 0.187312 | same | same | same |
| 600 | 0.002581 | -0.00205 | -0.79424 | same | same | same |
| 650 | 0.004603 | -0.00366 | 0.79569 | same | same | same |
| 700 | 0.00078 | 0.000688 | 0.88169 | same | same | same |

Table 3 shows that the Z values of batch b of Koleston Perfect 9/0 falls within the test statistic at all wavelengths. That is, Koleston Perfect shade 9/0 b has correctly passed the quality control according to the present invention, as it has been compared to the same color shade. Accordingly, the compared batches are statistically the same.

Example 3

Six variations of Illumina 7/81 have been produced. Each variant misses one key dye. Each variant has been subjected to the traditional dye-out test and to the test according to the present invention. The variations are described in Table 4 below.

Table 4: Varitions of Illumina 7/81, each variant missing one key dye

| Batch number | Missing Dye |
|---|---|
| DTF 980AF01/02 | Standard shade 7/81 |
| DTF 980BF01/02 | Toluene diamine sulfate (DTS) |
| DTF 980CF01/02 | Resorcinol (REOL) |
| DTF 980DF01/02 | 2,4-diaminophenoxyethanol (Lo-Blau) |
| DTF 980EF01/02 | m-aminophenol (MAP) |
| DTF 980FF01/02 | 4-amino-2-hydroxytoluene (AHT) |
| DTF 980GF01/02 | Hydroxyethyl-3,4-methylenedioxyaniline (Aminol) |

All variations correctly failed the quality control according to the present invention. The variation without Aminol incorrectly passed the traditional dye-out test by visual comparison with a "passed" sample, as it was judged to be sufficiently similar to the standard. The results are illustrated in Table 5 below. Further, Fig. 2a to 2f show graphically that each variant is out of spec as the A∞ values are outside of the 99% confidence limits for shade 7/81.

Table 5: Results of the two test methods regarding the varitions of Illumina 7/81

| Variation | Detected by dye out | Detected via UV/Vis |
|---|---|---|
| Without DTS | Yes | 99 % Confidence |
| Without Resorcinol | Yes | 99 % Confidence |
| Without Lo-Blau | Yes | 99 % Confidence |
| Without MAP | Yes | 99 % Confidence |

(continued)

| Variation | Detected by dye out | Detected via UV/Vis |
|---|---|---|
| Without AHT | Yes | 99 % Confidence |
| Without Aminol | No | 99 % Confidence |

Example 4

10 variations of Illumina 7/81 have been produced. Each variant contains +/- 1, 2, 5, 10, 20% DTS. Each variant has been subjected to the traditional dye-out test and to the test according to the present invention. The results are illustrated in Table 6 below, as well as in Fig. 3a to 3d.

Table 6: Results of the two test methods regarding the varitions of Illumina 7/81

| DTS levels versus FC target | Detected by dye out | Detected via UV/Vis |
|---|---|---|
| +/- 1 % | NO | No |
| +/- 2 % | NO | No |
| +/- 5 % | NO | 99 % Confidence |
| +/- 10 % | NO | 99 % Confidence |
| +/- 20 % | NO | 99 % Confidence |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A method for the quality control of a cosmetic composition which changes its electromagnetic spectral properties under application conditions from an initial state to an end state, wherein:

   - the composition is subjected to a measurement condition under which the change of electromagnetic spectral properties occurs over time,
   - the change of electromagnetic spectral properties is measured at least at two different points in time, where the electromagnetic spectral properties at an earlier point in time are different from the electromagnetic spectral properties at the later point in time,
   - at least one value of a parameter derivable from the measured change of electromagnetic spectral properties is determined and compared to a control value of the same parameter.

2. The method according to claim 1, wherein the change of electromagnetic spectral properties can be measured at a wavelength in the region of 200 nm to 700 nm.

3. The method according to any of the preceding claims, wherein the change of electromagnetic spectral properties from an initial state to an end state under application conditions occurs over a time range of from 1 second to 10 hour.

4. The method according to any of the preceding claims, wherein the change of electromagnetic spectral properties comprises a color change in the visible region of light.

5. The method according to any of the preceding claims, wherein the measurement condition is chosen such that the change of electromagnetic spectral properties is faster than under application conditions, preferably at least 2 times faster, more preferably at least 4 times, most preferably at least 6 times faster.

6. The method according to any of the preceding claims, wherein the cosmetic composition is an oxidative hair dyeing (coloring) composition comprising at least one oxidative dye precursor.

7. The method according to any of the preceding claims, wherein at least one catalyst is added to the cosmetic composition.

8. The method according to any of the preceding claims, wherein the change of spectral properties is measured at at least one wavelength within a timeframe of 1 to 20 minutes.

9. The method according to any of the preceding claims, wherein the at least one value of a parameter derivable from the measured change of electromagnetic spectral properties is selected from the group consisting of a kinetic value, an absorbance value, an inflection value or a mixture of two or more of those.

10. The method according to claim 9, wherein the absorbance value is absorbance at infinite reaction time.

11. The method according to any of the preceding claims, further comprising the step of comparing a determined value of the parameter derivable from the measured change of electromagnetic spectral properties with a pass/fail range for this value obtainable by:

a) determining the value of the at least one parameter derivable from the measured change of electromagnetic spectral properties for a population of a specific cosmetic composition with acceptable application properties;
b) determining a population mean, a standard deviation and a standard error for the value determined in step a);
c) defining a lower and an upper boundary between which the at least one value of the parameter derivable from the measured change of spectral properties to be controlled should range;
and determining whether the determined value of the parameter derivable from the measured change of electromagnetic spectral properties lies within this range or outside.

12. The method according to claim 11, wherein the population consists of at least 10 samples.

13. The method according to any of claims 11 or 12, wherein the comparison is carried out for at least the value of the reaction rate and the value of the absorbance at infinite time or the value of the reaction rate and the value of the inflection point or the value of the reaction rate and the value of the absorbance at infinite time and the value of the inflection point.

14. A method for the production of a cosmetic composition which changes its electromagnetic spectral properties under application conditions from an initial state to an end state, wherein the cosmetic composition is produced by mixing at least two constituents and at least one of the constituents or the mixture or both is subjected to a method as defined in any of claims 1 to 13.

15. The method according to claim 14, wherein the production is a continuous production.

## Fig. 1

Dye 9 Batch 1 mean

Batch 1 + 95%

Batch 2 – 95%

Batch 1-95%

Dye 9 Batch 2 mean

Batch 2 + 95%

## Fig. 2a

A ∞ values for shade 7-81 without DTS versus standard

7-81 upper

ML 7-81 WO DTS DTF0980037BF02 #1

7-81 lower

ML 7-81 WO DTS DTF0980037BF02 Rep.

# Fig. 2b

**A ∞ values for shade 7-81 without REOL versus standard**

| | |
|---|---|
| ——————— 7-81 upper | — — — — — ML 7-81 WO REOL DTF0980037CF01 #1 |
| ———···——— 7-81 lower | —— — ·· — ·· — ML 7-81 WO REOL DTF0980037CF01 #2 |

# Fig. 2c

**A ∞ values for shade 7-81 without LO-BLAU versus standard**

| | |
|---|---|
| ——————— 7-81 upper | — — — — — ML 7-81 WO LO-BLAU DTF0980037DF01 #1 |
| ———···——— 7-81 lower | —— — ·· — ·· — ML 7-81 WO LO-BLAU DTF0980037DF01 #2 |

## Fig. 2d

A ∞ values for shade 7-81 without AHT versus standard

| | |
|---|---|
| ———— 7-81 upper | — — — — ML 7-81 WO AHT DTF0980037FF02 #1 |
| ——···—— 7-81 lower | —·—·—·— ML 7-81 WO AHT DTF0980037FF02 #2 |

## Fig. 2e

A ∞ values for shade 7-81 without MAP versus standard

| | |
|---|---|
| ———— 7-81 upper | — — — — ML 7-81 WO MAP DTF0980037EF01 #1 |
| ——···—— 7-81 lower | —·—·—·— ML 7-81 WO MAP DTF0980037EF01 #2 |

## Fig. 2f

A∞ values for shade 7-81 without AMINOL versus standard

| | |
|---|---|
| ——————— 7-81 upper | — — — — — ML 7-81 WO AMINOL DTF0980037GF02 #1 |
| ——— ··· ——— 7-81 lower | — · · — · · — ML 7-81 WO AMINOL DTF0980037GF02 #2 |

## Fig. 3a

7/81 Rate constant +5%

| | | |
|---|---|---|
| ————— High | ——— ··· ——— Low | — — — — +5% |

## Fig. 3b

7/81 Infinity constant +5%

High          Low          +5%

## Fig. 3c

7/81 Rate constant -5%

High          Low          -5%

**Fig. 3d**

7/81 Infinity constant -5%

Legend: High, Low, -5%

Europäisches
Patentamt

European
Patent Office

Office européen
des brevets

# EUROPEAN SEARCH REPORT

Application Number

EP 15 16 6883

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 358 866 A2 (L'OREAL [FR]) 5 November 2003 (2003-11-05) * paragraphs [0001] - [0003], [0019], [0024], [0025], [0038] - [0040], [0073] - [0074]; claims 47-49 * | 1-15 | INV. G01N21/27 G01N21/78 A61K8/00 A61Q5/00 A45D44/00 G01J3/46 A61B5/00 |
| X | US 5 662 890 A (PUNTO LOUIS L [US] ET AL) 2 September 1997 (1997-09-02) * column 1, lines 11-17 * * column 4, lines 4-23 * * column 6, lines 15-22 * * column 7, line 23 - column 12, line 8; figures 4,5 * | 1-4,9, 11-15 | |
| X | US 2013/149358 A1 (COLACO ALLWYN [US] ET AL) 13 June 2013 (2013-06-13) * paragraphs [0063] - [0068]; figure 1; example I * | 1,2,4-9, 14 | |
| X | US 2009/081143 A1 (MAMMONE THOMAS [US] ET AL) 26 March 2009 (2009-03-26) * paragraphs [0001], [0012], [0013], [0017], [0018], [0040] - [0043]; figures 4,5; example 5 * | 1,2,4,9, 11-15 | TECHNICAL FIELDS SEARCHED (IPC) G01N G01J A61K A61Q A45D A61B |

The present search report has been drawn up for all claims

| Place of search | Date of completion of the search | Examiner |
|---|---|---|
| Munich | 24 November 2015 | Duijs, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 16 6883

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2015

| Patent document cited in search report | | | Publication date | Patent family member(s) | | | Publication date |
|---|---|---|---|---|---|---|---|
| EP | 1358866 | A2 | 05-11-2003 | CN | 1454586 | A | 12-11-2003 |
| | | | | EP | 1358866 | A2 | 05-11-2003 |
| | | | | JP | 4189877 | B2 | 03-12-2008 |
| | | | | JP | 2003321327 | A | 11-11-2003 |
| | | | | US | 2004018163 | A1 | 29-01-2004 |
| | | | | US | 2011110990 | A1 | 12-05-2011 |
| US | 5662890 | A | 02-09-1997 | AU | 684483 | B2 | 18-12-1997 |
| | | | | CA | 2149861 | A1 | 09-06-1994 |
| | | | | EP | 0746301 | A1 | 11-12-1996 |
| | | | | JP | 3655305 | B2 | 02-06-2005 |
| | | | | JP | H08503709 | A | 23-04-1996 |
| | | | | US | 5662890 | A | 02-09-1997 |
| | | | | WO | 9412146 | A1 | 09-06-1994 |
| US | 2013149358 | A1 | 13-06-2013 | AR | 089049 | A1 | 23-07-2014 |
| | | | | CA | 2854801 | A1 | 13-06-2013 |
| | | | | CN | 103987375 | A | 13-08-2014 |
| | | | | EP | 2787963 | A2 | 15-10-2014 |
| | | | | TW | 201323009 | A | 16-06-2013 |
| | | | | US | 2013149358 | A1 | 13-06-2013 |
| | | | | US | 2014044762 | A1 | 13-02-2014 |
| | | | | WO | 2013085577 | A2 | 13-06-2013 |
| US | 2009081143 | A1 | 26-03-2009 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1942343 A

- WO 08124178 A1

**Non-patent literature cited in the description**

- International Cosmetics Ingredient Dictionary and Handbook. The Cosmetics, Toiletry, and Fragrance Association, vol. 2